(19) Europäisches Patentamt
European Patent Office
Office européen des brevets

(11) **EP 4 446 728 A1**

(12) **EUROPEAN PATENT APPLICATION**
published in accordance with Art. 153(4) EPC

(43) Date of publication:
**16.10.2024 Bulletin 2024/42**

(21) Application number: **22904127.2**

(22) Date of filing: **01.12.2022**

(51) International Patent Classification (IPC):
**G01N 21/01** (2006.01)    **G01N 21/3504** (2014.01)

(52) Cooperative Patent Classification (CPC):
**G01N 21/01; G01N 21/3504**

(86) International application number:
**PCT/JP2022/044356**

(87) International publication number:
**WO 2023/106196 (15.06.2023 Gazette 2023/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.12.2021 JP 2021200845**

(71) Applicant: HORIBA, Ltd.
**Kyoto-shi, Kyoto 601-8510 (JP)**

(72) Inventors:
• **TSUKATANI, Kosuke**
**Kyoto-shi, Kyoto 601-8510 (JP)**

• **HOTTA, Kohei**
**Kyoto-shi, Kyoto 601-8510 (JP)**
• **YAMAMOTO, Tomomi**
**Kyoto-shi, Kyoto 601-8510 (JP)**
• **IDO, Takuya**
**Kyoto-shi, Kyoto 601-8510 (JP)**
• **SHIBUYA, Kyoji**
**Kyoto-shi, Kyoto 601-8510 (JP)**

(74) Representative: **Müller Hoffmann & Partner Patentanwälte mbB**
**St.-Martin-Straße 58**
**81541 München (DE)**

(54) **ANALYSIS DEVICE AND ANALYSIS METHOD**

(57)    An analysis device that irradiates a measurement cell into which a sample has been introduced with light, detects light having passed through the measurement cell, and analyzes a component to be measured contained in the sample, the analysis device includes: two or more laser light sources selected from a first laser light source that is a quantum cascade laser, a second laser light source that is an interband cascade laser, and a third laser light source that is a semiconductor laser other than the quantum cascade laser and the interband cascade laser; a photodetector that detects light emitted from each of the laser light sources and having passed through the measurement cell; and a light source control unit that causes the laser light sources to perform pulse oscillation at different timings.

FIG.1

**Description**

Technical Field

The present invention relates to an analysis device and an analysis method used for, for example, component analysis of gas.

Background Art

Conventionally, as an analysis device using a light source such as a laser, in order to measure a plurality of components included in a sample, an analysis device including a measurement cell in which the sample is accommodated, a plurality of laser light sources which are light sources for irradiating the measurement cell with laser light, and a photodetector for detecting light transmitted through the measurement cell is known. As such an analysis device, for example, Patent Literature 1 discloses a device in which drive voltages of a plurality of quantum cascade lasers as laser light sources are controlled so that oscillation wavelengths of the respective quantum cascade lasers correspond to different components to be measured, and pulse oscillation is performed at different timings, so that the plurality of components to be measured are analyzed in a short time by a single analysis device.

Citation List

Patent Literature

Patent Literature 1: JP 2019-066477 A

Summary of Invention

Technical Problem

However, since the above-described analysis device uses the quantum cascade laser as the plurality of laser light sources, the components that can be analyzed by the analysis device are limited to those of which the peaks of the absorption spectra are included in the oscillation wavelength range of the quantum cascade laser.
The present invention has been made in view of the above-described problems, and a main object thereof is to enable analysis of many types of components in a short time in an analysis device using a laser light source.

Solution to Problem

That is, according to the present invention, an analysis device that irradiates a measurement cell into which a sample has been introduced with light, detects light having passed through the measurement cell, and analyzes a component to be measured contained in the sample, the analysis device includes: two or more laser light sources selected from a first laser light source that is a quantum cascade laser, a second laser light source that is an interband cascade laser, and a third laser light source that is a semiconductor laser other than the quantum cascade laser and the interband cascade laser; a photodetector that detects light emitted from each of the laser light sources and having passed through the measurement cell; and a light source control unit that causes the laser light sources to perform pulse oscillation at different timings.
According to the present invention, since a plurality of different types of semiconductor lasers are used as the laser light sources, a range of selection of the oscillation wavelengths of the laser light can be increased as compared with a case where a plurality of semiconductor lasers of the same type is used as the laser light sources, and a larger variety of components can be analyzed. In addition, since the laser light sources are caused to perform pulse oscillation at different timings from each other, measurement by another laser light source becomes possible while a pulse of a certain laser light source is turned off, and a plurality of components can be simultaneously analyzed by a single photodetector. As a result, the analysis time can be shortened, and the power consumption can be reduced as compared with the case where each laser light source performs continuous wave (CW) oscillation. In particular, the quantum cascade laser has high power consumption due to its characteristics, and when the CW oscillation is performed, a calorific value is large, whereby a heat discharge mechanism becomes large, leading to an increase in device size and cost.
In the present specification, the quantum cascade laser is a semiconductor laser using intersubband transition by a multistage quantum well structure, and can mainly oscillate mid-infrared light between 4 and 20 $\mu$m. The interband cascade laser is a semiconductor laser using interband transition of a multistage PN junction by a quantum well structure, and can mainly oscillate mid-infrared light between 3 and 5 $\mu$m. The semiconductor laser other than the quantum cascade laser and the interband cascade laser is a semiconductor laser using interband transition of a single PN junction by a quantum well structure, and can mainly oscillate ultraviolet light, visible light, or near infrared light between 0.3 and 3 $\mu$m.

The analysis device is preferably configured such that the light source control unit causes the laser light sources to perform pulse oscillation at the same oscillation cycle.

In this way, since a light intensity signal corresponding to each laser light source is sequentially output from the photodetector at the same time difference in each oscillation cycle, signal processing is facilitated, and each component can be analyzed in a shorter time.

The analysis device is preferably configured such that the light emitted from each of the laser light sources and having passed through the measurement cell is detected by a smaller number of the photodetectors than the number of the laser light sources, and more preferably configured such that the light emitted from each of the laser light sources and having passed through the measurement cell is detected by the single photodetector.

In this way, since the photodetector can be shared among the plurality of laser light sources, the device configuration can be simplified, and the analysis device can be reduced in size.

As a specific aspect of the analysis device, an analysis device can be exemplified that is configured such that the laser light sources emit laser lights having oscillation wavelengths respectively corresponding to different components to be measured.

In order to enable a single photodetector to detect light from a plurality of laser light sources, it is preferable to have sensitivity to the oscillation wavelength of each of the laser light sources, and for example, it is preferable to have sensitivity over a wide wavelength range between 2 and 10 $\mu$m or more. In this case, in order to increase the response speed of the photodetector and enable a small number of photodetectors to analyze multiple components in a short time, the photodetector preferably uses a quantum photoelectric element, and particularly preferably uses InAsSb or HgCdTe as a detection element.

Incidentally, in this type of analysis device, in order to improve analysis accuracy, the oscillation wavelength of the laser light output from the laser light source is often modulated (wavelength sweep) around the peak of the component to be measured by changing the drive current and the drive voltage of the laser light source at a predetermined frequency. Patent Literature 1 discloses that an oscillation wavelength of a laser light source is modulated by changing a base current or a voltage (a value equal to or less than a threshold for pulse oscillation) for modulation different from a constant pulse current or pulse voltage for pulse oscillation of a quantum cascade laser as a laser light source at a predetermined frequency.

The analysis device preferably includes the first laser light source and the second laser light source or the third laser light source, and is configured such that the light source control unit modulates an oscillation wavelength of the first laser light source by changing a base current or a base voltage for modulation of the first laser light source at a predetermined frequency, and modulates an oscillation wavelength of the second laser light source or the third laser light source by changing a base current or a base voltage for modulation of the second laser light source or the third laser light source at a predetermined frequency.

As described above, by driving the second laser light source which is an interband cascade laser or the third laser light source which is a semiconductor laser other than the interband cascade laser by the same driving method as the first laser light source which is a quantum cascade laser, it is possible to generate a temperature change of the element of each laser light source and sufficiently modulate the oscillation wavelength.

On the other hand, as a result of intensive studies by the present inventors, it has been found that when a driving method of a quantum cascade laser in which a base current for modulation or the like is changed at a predetermined frequency together with a constant pulse current for pulse oscillation or the like is applied to an interband cascade laser or a semiconductor laser other than the interband cascade laser, there is a rare case where sufficient modulation cannot be performed on an absorption linewidth of an optical absorption spectrum of a component to be measured.

As a result of further intensive studies by the present inventors, it has been found that such a case rarely occurs because the quantum cascade laser has a relatively high drive power due to a difference in the operation principle of various laser light sources, and thus has a large effect on the wavelength modulation of the temperature change of the element due to the change in the base current or the like, whereas the wavelength modulation of the interband cascade laser or a semiconductor laser other than the interband cascade laser has a relatively low drive power, and thus has a small effect on the wavelength modulation of the wavelength change of the temperature change of the element due to the change in the base current or the like. In addition, the present inventors have found that, in an interband cascade laser or a semiconductor laser other than the interband cascade laser, an effect of a change in carrier density of an element due to a change in a drive current or the like for laser oscillation on wavelength modulation is relatively large, and therefore a range of wavelength modulation can be extended by leveraging this effect.

Therefore under some condition, the analysis device includes the first laser light source and the second laser light source or the third laser light source, and the light source control unit may be configured to modulate an oscillation wavelength of the first laser light source by changing a base current or a base voltage for modulation of the first laser light source at a predetermined frequency, and modulate an oscillation wavelength of the second laser light source or the third laser light source by changing a peak value of a pulse current or a pulse voltage for pulse oscillation of the second laser light source or the third laser light source at a predetermined frequency.

Also in this case, the oscillation wavelength of the first laser light source which is a quantum cascade laser, and the oscillation wavelength of the second laser light source which is an interband cascade laser or the third laser light source which is a semiconductor laser other than the interband cascade laser can be modulated. That is, for the first laser light source in which the effect of wavelength modulation due to the temperature change of the element is dominant due to its characteristics, the oscillation wavelength can be modulated by changing the base value such as the drive current. On the other hand, for the second laser light source or the third laser light source in which the effect of wavelength modulation due to the change in carrier density of the element is dominant due to its characteristics, the oscillation wavelength can be modulated by changing the peak value of the drive current or the like, that is, the pulse current or the like.

As a specific aspect of the analysis device, it is preferable that the component to be measured contains at least HCl and/or HF, and the light source control unit modulates an oscillation wavelength of any one of the laser light sources so as to correspond to an optical absorption spectrum of HCl and modulates an oscillation wavelength of any one of the laser light sources so as to correspond to an optical absorption spectrum of HF.

In this way, it is possible to accurately analyze HCl and/or HF that cannot be accurately analyzed when only a quantum cascade laser is used as a laser light source.

It is preferable that the analysis device includes a concentration calculation unit that calculates a concentration of the component to be measured on the basis of an output signal of the photodetector, in which the concentration calculation unit, when the concentration of HCl is measured, calculates the concentration based on absorption of HCl of 3.30 $\mu$m or more and 3.64 $\mu$m or less, and when the concentration of HF is measured, calculates the concentration based on absorption of HF of 2.39 $\mu$m or more and 2.65 $\mu$m or less. Here, the second laser light source emits laser light having an oscillation wavelength including a wavelength of 3.30 $\mu$m or more and 3.64 $\mu$m or less. On the other hand, the third laser light source emits laser light having an oscillation wavelength including a wavelength of 2.39 $\mu$m or more and 2.65 $\mu$m or less.

An analysis method according to the present invention irradiates a measurement cell into which a sample has been introduced with light, detects light having passed through the measurement cell, and analyzes a component to be measured contained in the sample, the analysis device including: causing two or more laser light sources selected from a first laser light source that is a quantum cascade laser, a second laser light source that is an interband cascade laser, and a third laser light source that is a semiconductor laser other than the quantum cascade laser and the interband cascade laser to perform pulse oscillation at different timings; and detecting light emitted from each of the laser light sources and having passed through the measurement cell by a photodetector.

With such an analysis method, it is possible to achieve operational effects similar to those of the analysis device of the present invention described above.

Advantageous Effects of Invention

According to the present invention described above, it is possible to analyze many types of components in a short time in an analysis device using a laser light source.

Brief Description of Drawings

Fig. 1 is an overall schematic view of an analysis device according to an embodiment of the present invention.
Fig. 2 is a functional block diagram of a signal processing device according to the embodiment.
Fig. 3 is a diagram showing a drive current (voltage) and a modulation signal of the first laser light source in the embodiment.
Fig. 4 is a diagram showing a drive current (voltage) and a modulation signal of the second laser light source in the embodiment.
Fig. 5 is a diagram showing a drive current (voltage) and a modulation signal of the third laser light source in the embodiment.
Fig. 6 is a schematic view illustrating a method of modulating a laser oscillation wavelength in the embodiment.
Fig. 7 is a schematic view illustrating a measurement principle by pseudo continuous oscillation.
Fig. 8 is a schematic view showing an example of pulse oscillation timings and light intensity signals of a plurality of laser light sources in the embodiment.
Fig. 9 is a schematic view illustrating a configuration of a signal separation unit of the embodiment.

Fig. 10 is a diagram illustrating an example of a sample-and-hold circuit according to the embodiment.

Fig. 11 is a time-series graph illustrating an example of an oscillation wavelength, light intensity I(t), logarithmic intensity L(t), a feature signal $F_i(t)$, and a correlation value Si in the embodiment.

Fig. 12 is a diagram illustrating a conceptual diagram of concentration calculation using a single correlation value and a sample correlation value according to the embodiment. Description of Embodiments

Hereinafter, an analysis device 100 according to an embodiment of the present invention will be described with reference to the drawings.

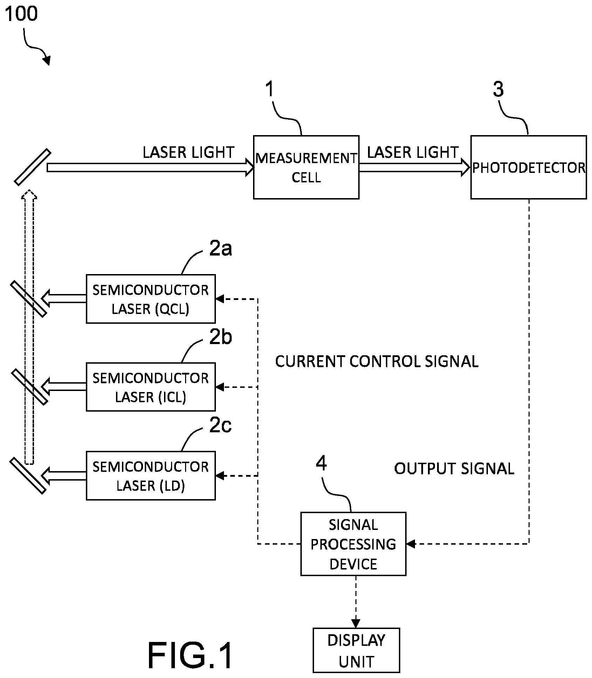

The analysis device 100 is a concentration measurement device that measures the concentration of one or a plurality of types of components to be measured (herein, for example, $CO$, $CO_2$, $N_2O$, $NO$, $NO_2$, $H_2O$, $SO_2$, $CH_4$, $NH_3$, $HF$, $HCl$, $H_2S$, $HBr$, $HCN$, or the like) contained in sample gas such as exhaust gas, and includes, as shown in Fig. 1, a measurement cell 1 into which sample gas is introduced, a plurality of laser light sources 2 that irradiate the measurement cell 1 with laser light, a photodetector 3 that is provided on an optical path of the laser light transmitted through the measurement cell 1 and receives the laser light, and a signal processing device 4 that receives a light intensity signal that is an output signal of the photodetector 3 and calculates the concentration of the component to be measured based on the value.

The cell 1 is made of a transparent material such as quartz, calcium fluoride, or barium fluoride, which hardly absorbs light in the absorption wavelength band of the component to be measured, and has a light entrance/exit port. Although not illustrated, the cell 1 is provided with an inlet port for introducing gas thereinto and an outlet port for discharging gas from inside, and the sample gas is introduced into the cell 1 from the inlet port.

The laser light source 2 can modulate (change) the oscillation wavelength by a given current (or voltage). The analysis device 100 of the present embodiment includes a plurality of types of semiconductor lasers having different oscillation wavelength ranges as the plurality of laser light sources 2, and specifically includes a first laser light source 2a that is a quantum cascade laser (QCL), a second laser light source 2b that is an interband cascade laser (ICL), and a third laser light source 2c that is a semiconductor laser other than the quantum cascade laser and the interband cascade laser.

The quantum cascade laser as the first laser light source 2a is a semiconductor laser using an intersubband transition by a multistage quantum well structure, and oscillates laser light of a specific wavelength in a wavelength range of about 4 $\mu$m to about 20 $\mu$m, and the interband cascade laser as the second laser light source 2b is a semiconductor laser using an interband transition of a multistage PN junction by a quantum well structure, and oscillates laser light of a specific wavelength in a wavelength range of about 3 $\mu$m to about 5 $\mu$m. The third laser light source 2c is a semiconductor laser using an interband transition of a single PN junction by a quantum well structure, and is a semiconductor laser capable of oscillating mainly ultraviolet light, visible light, and near infrared light between 0.3 and 3 $\mu$m. The third laser light source 2c of the present embodiment is a near-infrared laser diode that oscillates laser light having a specific wavelength in a wavelength range of about 1 $\mu$m to about 3 $\mu$m.

The photodetector 3 uses a quantum photoelectric element having good responsiveness, and in the present embodiment, InAsSb is used as a detection element. Note that the detection element is not limited thereto, and for example, HgCdTe, InGaAs, PbSe, or the like may be used. In the present embodiment, InAsSb having sensitivity in a wide wavelength range is used as a detection element, so that light emitted from the plurality of laser light sources 2a to 2c and passing through the measurement cell 1 is detected by a single (common) photodetector 3. When the intensity of the laser light having passed through the measurement cell 1 is high and the linearity of the photodetector 3 affects the measurement, a light intensity adjustment mechanism such as a light attenuator may be provided on the optical path of the laser light.

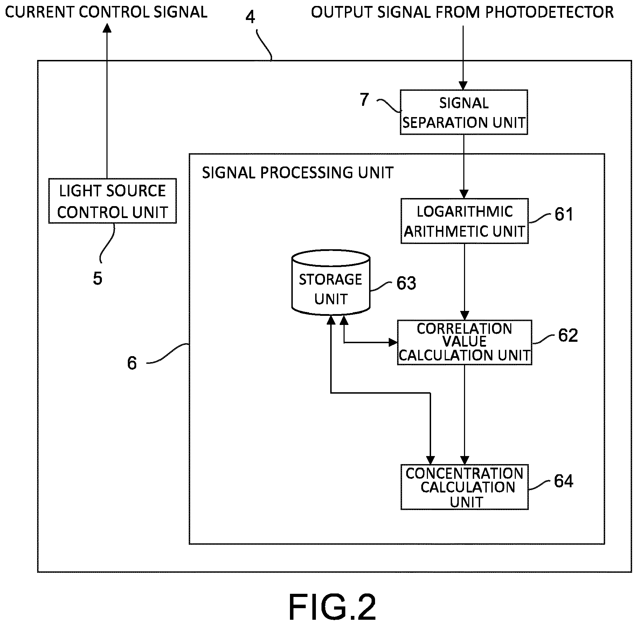

The signal processing device 4 includes an analog electric circuit including a buffer, an amplifier, and the like, a digital electric circuit including a CPU, a memory, and the like, and an AD converter, a DA converter, and the like that intervene between the analog and digital electric circuits. Then, the signal processing device 4 functions as a light source control unit 5 that controls the output of each laser light source 2, a signal separation unit 7 that separates the signal for each laser light source 2 from the light intensity signal obtained by the photodetector 3, and a signal processing unit 6 that receives the signal for each laser light source 2 separated by the signal separation unit 7, and performs arithmetic processing on the value to calculate the concentration of the component to be measured, as illustrated in Fig. 2, by cooperation of the CPU and its peripheral devices according to a predetermined program stored in a predetermined area of the memory.

Each unit will be described in detail below.

The light source control unit 5 causes each of the plurality of laser light sources 2a to 2c to perform pulse oscillation and modulates the oscillation wavelength of the laser light at a predetermined frequency. In addition, the light source control unit 5 performs control so that the plurality of laser light sources 2a to 2c have oscillation wavelengths corresponding to different components to be measured, and performs pulse oscillation so that the oscillation cycles are the same as each other and the oscillation timings are different from each other.

Specifically, the light source control unit 5 controls the current source (or the voltage source) of each of the laser light sources 2a to 2c by outputting a current (or voltage) control signal to set the drive current (drive voltage) of the current source (or the voltage source) to be equal to or more than a predetermined threshold value for causing pulse oscillation. The light source control unit 5 causes each of the laser light sources 2a to 2c to perform pseudo continuous oscillation (pseudo CW) by pulse oscillation with a predetermined pulse width (For example, between 10 and 100 ns, and a duty ratio of 5%) repeated at a predetermined cycle (for example, between 0.5 and 5 MHz).

Here, the light source control unit 5 of the present embodiment is configured to modulate the oscillation wavelengths of the laser light sources 2a to 2c by different control methods.

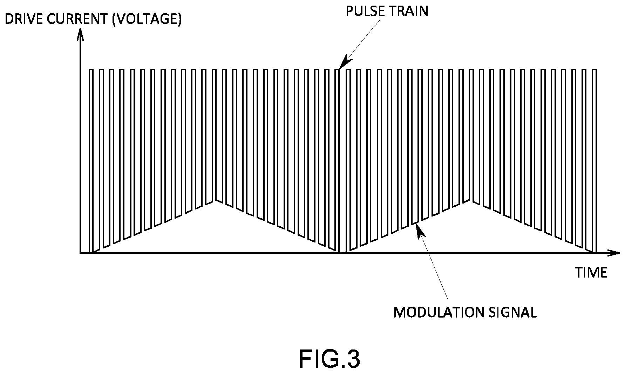

Specifically, as illustrated in Fig. 3, the light source control unit 5 is configured to control a current source (or a voltage source) of the first laser light source 2a to change a base current (base voltage) less than an oscillation threshold for wavelength modulation at a predetermined frequency, thereby generating a temperature change of the element and sweeping the oscillation wavelength of the first laser light source 2a. Here, the light source control unit 5 sets the peak value of the pulse current (pulse voltage) for pulse oscillation of the current source (or voltage source) of the first laser light source 2a to a constant value without changing the peak value.

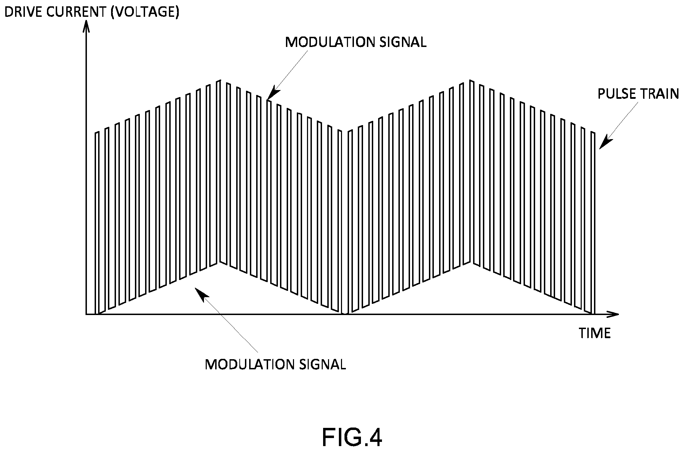

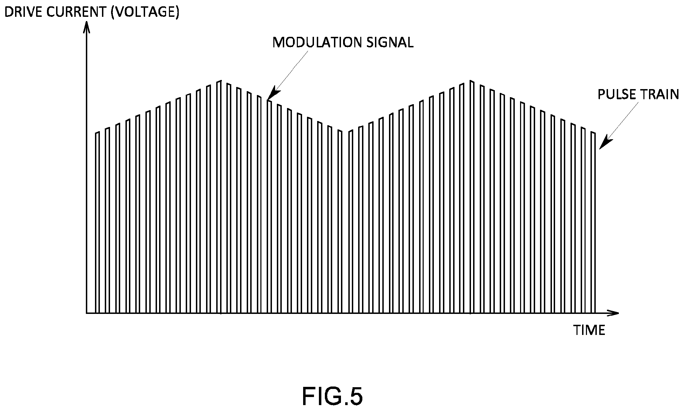

As illustrated in Figs. 4 and 5, the light source control unit 5 is configured to control current sources (or voltage sources) of the second laser light source 2b and the third laser light source 2c, respectively, to change a peak value of a pulse current (pulse voltage) equal to or higher than an oscillation threshold for pulse oscillation at a predetermined frequency, thereby generating a change in carrier density of the element, and sweeping the oscillation wavelengths of the second laser light source 2b and the third laser light source 2c. Here, as illustrated in Fig. 4, the light source control unit 5 changes the base current (base voltage) of the current source (or voltage source) of the second laser light source 2b at a predetermined frequency. As illustrated in Fig. 5, the light source control unit 5 sets the base current of the current source (or voltage source) of the third laser light source 2c to a constant value or 0 without changing the base current.

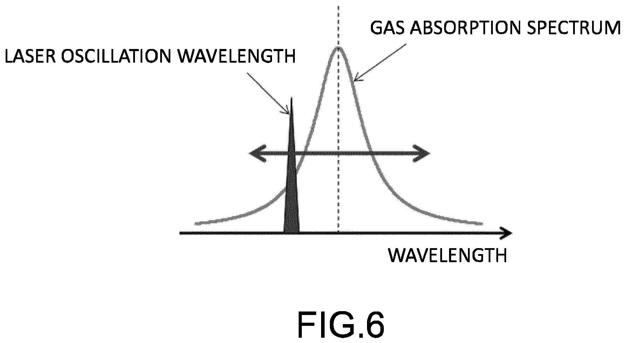

As illustrated in Fig. 6, the oscillation wavelength of the laser light in each of the laser light sources 2a to 2c is modulated around the peak of the optical absorption spectrum of the component to be measured. The modulation signal that changes the drive current is a signal that changes in a triangular waveform, a sawtooth waveform, or a sine waveform and has a frequency of, for example, between 0.1 and 10 kHz. Figs. 3 to 5 illustrate examples in which the modulated signal changes in a triangular waveform.

In addition, the light source control unit 5 controls the plurality of laser light sources 2a to 2c to have oscillation wavelengths corresponding to different components to be measured.

Here, when the component to be measured is CO, $CO_2$, $N_2O$, NO, $NO_2$, $H_2O$, $SO_2$, $CH_4$, or $NH_3$, the light source control unit 5 modulates the oscillation wavelength of the first laser light source 2a so as to correspond to the optical absorption spectrum of each component.

When the component to be measured is HCl (hydrogen chloride), the light source control unit 5 modulates the oscillation wavelength of the second laser light source 2b so as to correspond to the optical absorption spectrum of HCl. Specifically, the light source control unit 5 performs modulation so that the wavelength modulation range of the laser light of the second laser light source 2b preferably includes a wavelength of 3.30 μm or more and 3.64 μm or less, and more preferably any one of wavelengths of 3.3355 μm, 3.3546 μm, 3.3746 μm, 3.5728 μm, and 3.6026 μm. By modulating in this manner, the interference influence of $H_2O$ (water) and/or $CH_4$ (methane) can be reduced, and the measurement accuracy of the concentration of low-concentration HCl can be improved.

When the component to be measured is HF (hydrogen fluoride), the light source control unit 5 modulates the oscillation wavelength of the third laser light source 2c so as to correspond to the optical absorption spectrum of HF. Specifically, the light source control unit 5 performs modulation so that the wavelength modulation range of the laser light of the second laser light source 2c preferably includes a wavelength of 2.39 μm or more and 2.65 μm or less, and more preferably any one of wavelengths of 2.3958 μm, 2.4138 μm, 2.4331 μm, 2.4538 μm, and 2.6398 μm. By modulating in this manner, the interference influence of $CO_2$ (carbon dioxide), CO (carbon monoxide), $H_2O$ (water), and/or $CH_4$ (methane) can be reduced, and the measurement accuracy of the concentration of HF having a low concentration can be improved.

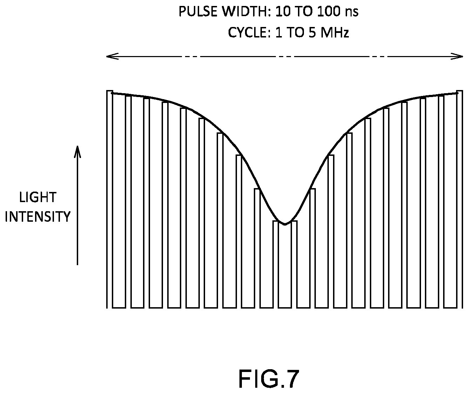

The light intensity signal obtained by the photodetector 3 by causing one laser light source 2 to perform pseudo continuous oscillation as described above is as illustrated in Fig. 7. Thus, the optical absorption spectrum (absorption signal) can be acquired in the entire pulse train.

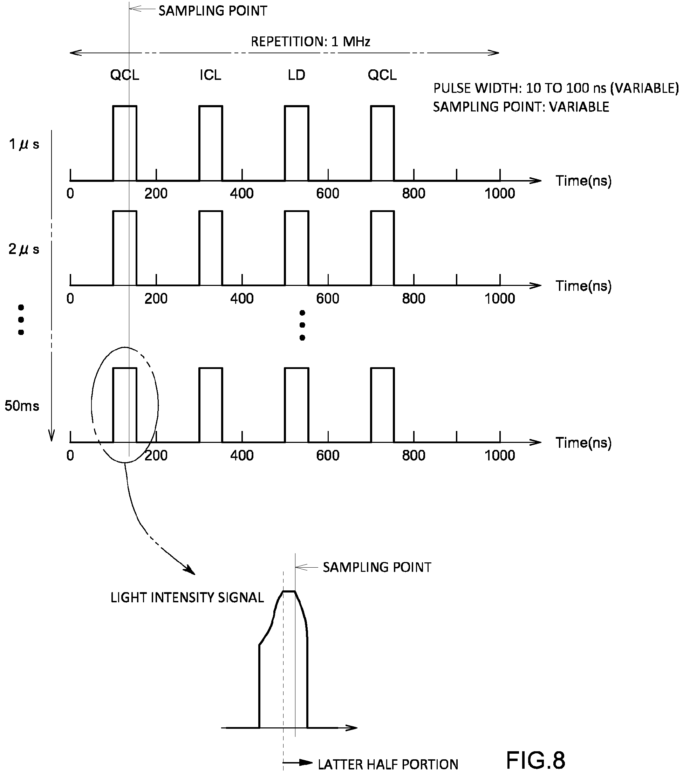

In addition, the light source control unit 5 pulse-oscillates each of the laser light sources 2a to 2c at different timings. Specifically, as illustrated in Fig. 8, each of the laser light sources 2a to 2c sequentially performs pulse oscillation, and one pulse of each of the other laser light sources 2 is included in one cycle of pulse oscillation in one laser light source 2. That is, one pulse of each of the other laser light sources 2 is included in adjacent pulses of one laser light source 2. At this time, the pulses of the plurality of laser light sources 2 are oscillated so as not to overlap each other.

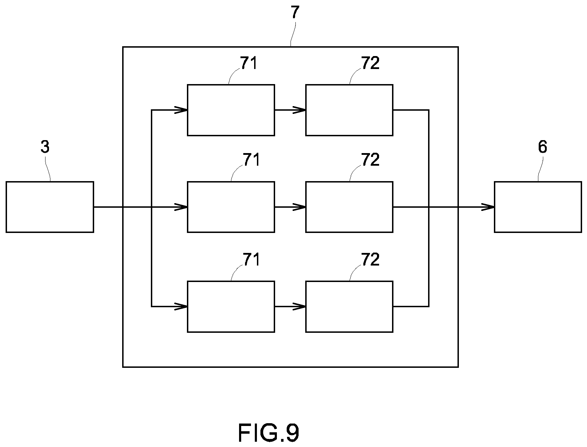

The signal separation unit 7 separates the signal of each of the plurality of laser light sources 2a to 2c from the light intensity signal obtained by the photodetector 3. As illustrated in Fig. 9, the signal separation unit 7 of the present embodiment includes a plurality of sample-and-hold circuits 71 provided to correspond to the plurality of laser light sources 2a to 2c, respectively, and an AD converter 72 that digitally converts the light intensity signals separated by the sample-and-hold circuits 71. Note that the sample-and-hold circuit 71 and the AD converter 72 may be common to the plurality of laser light sources 2a to 2c.

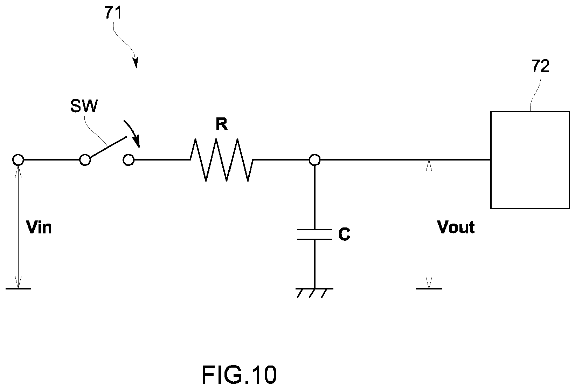

The sample-and-hold circuit 71 separates and holds a signal of the corresponding laser light source 2 from a light intensity signal of the photodetector 3 at a timing synchronized with a pulse oscillation timing of the laser light source 2 by a sampling signal synchronized with a current (or voltage) control signal of the corresponding laser light source 2. An example of the sample-and-hold circuit 71 is illustrated in Fig. 10, but the present invention is not limited thereto. Here, the sample-and-hold circuit 71 is configured to separate and hold a signal corresponding to the latter half portion of the pulse oscillation of the laser light source 2. Specifically, the open/close timing of a switch SW of the sample-and-hold circuit 71 holds a signal corresponding to the latter half portion of the pulse oscillation in synchronization with the timing of the pulse oscillation of the laser light source 2. In addition, as illustrated in Fig. 8, the sample-and-hold circuit 71 separates signals at a predetermined sampling point in the latter half portion (for example, at the time point between 80 and 90 ns). By collecting the plurality of signals of the laser light sources 2 separated by the signal separation unit 7, one optical absorption spectrum is obtained, and a spectrum having better wavelength resolution than the optical absorption spectrum obtained when one laser light source 2 is caused to perform pseudo continuous oscillation can be obtained. A plurality of optical absorption spectra obtained for each laser light source 2 may be time-averaged and used. Here, since the signal corresponding to a part of the pulse oscillation is separated by the sample-and-hold circuit 71, the AD converter 72 may have a slow processing speed.

Using the absorption spectrum of each laser light source 2 separated by the signal separation unit 7 as described above, the signal processing unit 6 calculates the concentration of the component to be measured corresponding to each laser light source 2.

The signal processing unit 6 includes a logarithmic arithmetic unit 61, a correlation value calculation unit 62, a storage unit 63, a concentration calculation unit 64, and the like.

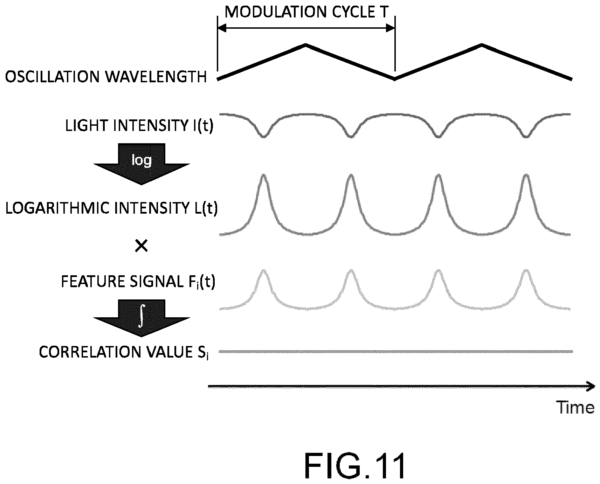

The logarithmic arithmetic unit 61 performs logarithmic arithmetic on a light intensity signal which is an output signal of the photodetector 3. The function I(t) indicating the time-dependent change in the light intensity signal obtained by the photodetector 3 is expressed as "light intensity I(t)" in Fig. 11, and becomes "logarithmic intensity L(t)" in Fig. 11 by performing logarithmic arithmetic.

The correlation value calculation unit 62 calculates a correlation value between an intensity-related signal related to the intensity of the sample light and each of a plurality of predetermined feature signals. The feature signal is a signal for extracting a waveform feature of the intensity-related signal by correlating with the intensity-related signal. As the feature signal, for example, various signals corresponding to waveform features to be extracted from a sine wave signal or other intensity-related signals can be used.

Hereinafter, an example of a case where a feature signal other than the sine wave signal is used will be described. The correlation value calculation unit 62 calculates a correlation value of each of the intensity-related signal related to the intensity of the sample light and a plurality of feature signals in which a correlation different from that of the sine wave signal (sine function) is obtained with respect to the intensity-related signal. Here, the correlation value calculation unit 62 uses the light intensity signal (logarithmic intensity L(t)) subjected to logarithmic arithmetic as the intensity-related signal.

In addition, the correlation value calculation unit 62 calculates a plurality of sample correlation values $S_i(t)$ by the following equations (Math 1) using the feature signals $F_i(t)$ (i = 1, 2, ..., n) of the number equal to or larger than the sum of the number of types of components to be measured and the number of types of interference components. Note that T in Math 1 is a modulation cycle.

[Math 1]

$$S_i = \int_0^T L(t) \cdot F_i(t)\, dt \quad (i = 1, 2, \cdots, n)$$

$$R_i = \int_0^T L_0(t) \cdot F_i(t)\, dt \quad (i = 1, 2, \cdots, n)$$

$$S_i{'} = S_i - R_i$$

When calculating the sample correlation value, the correlation value calculation unit 62 obtains a correlation value Si between the intensity-related signal L(t) of the sample light and the plurality of feature signals $F_i(t)$ as in the above equation (Math 1). In addition, the correlation value calculation unit 62 obtains a reference correlation value $R_i$, which is a correlation value between the intensity-related signal $L_0(t)$ of the reference light and the plurality of feature

signals $F_i(t)$, as in the above equation (Math 1). Then, the correlation value calculation unit 62 calculates a sample correlation value $S_i'$ obtained by subtracting the reference correlation value Ri from the correlation value Si as in the above formula (Math 1). As a result, the offset included in the sample correlation value is removed, the correlation value is proportional to the concentration of the component to be measured and the interference component, and the measurement error can be reduced. Note that the reference correlation value may not be subtracted.

Here, the acquisition timing of the reference light is the same as that of the sample light, before and after the measurement, or any timing. The intensity-related signal or the reference correlation value of the reference light may be acquired in advance and stored in the storage unit 63. In addition, as a method for simultaneously acquiring the reference light, for example, it is conceivable that two photodetectors 3 are provided, the modulated light from the laser light source 2 is split by a beam splitter or the like, of which one is for sample light measurement and the other is for reference light measurement.

In the present embodiment, the correlation value calculation unit 62 uses, as the plurality of feature signals $F_i(t)$, a function that more easily captures waveform features of the logarithmic intensity L(t) than a sine function. In the case of a sample gas containing a component to be measured (for example, $SO_2$) and one interference component (for example, $H_2O$), it is conceivable to use two or more feature signals $F_1(t)$ and $F_2(t)$, and it is conceivable to use, for example, a function based on a Lorentzian function close to a form of an absorption spectrum and a differential function of a function based on the Lorentzian function as the two feature signals $F_1(t)$ and $F_2(t)$. As the feature signal, instead of the function based on the Lorentzian function, a function based on the Voigt function, a function based on the Gaussian function, or the like can also be used. By using such a function for the feature signal, a larger correlation value can be obtained than when the sine function is used, and measurement accuracy can be improved.

Here, it is desirable to adjust the offset of the feature signal so that the DC component is removed, that is, the offset becomes zero when integrated by the modulation cycle. In this way, it is possible to remove the influence of when the offset is added to the intensity-related signal due to the variation in the light intensity. Instead of removing the DC component of the feature signal, the DC component of the intensity-related signal may be removed, or the DC component of both the feature signal and the intensity-related signal may be removed. In addition, an actual measurement value of the absorption signal of the component to be measured and/or the interference component, or an imitation thereof may be used as the feature signal.

Note that by setting the two feature signals $F_1(t)$ and $F_2(t)$ to an orthogonal function sequence or a function sequence close to an orthogonal function sequence orthogonal to each other, the feature of the logarithmic intensity L(t) can be more efficiently extracted, and the concentration obtained by simultaneous equations described later can be made accurate.

The storage unit 63 stores a single correlation value that is a correlation value per unit concentration of each of the component to be measured and each of the interference components obtained from each of the intensity-related signals and the plurality of feature signals $F_i(t)$ in a case where the component to be measured and each of the interference components exist alone. The plurality of feature signals $F_i(t)$ used to obtain the single correlation value is the same as the plurality of feature signals $F_i(t)$ used in the correlation value calculation unit 62.

Here, when storing the single correlation value, the storage unit 63 desirably stores the single correlation value corrected to be converted per unit concentration after subtracting the reference correlation value from the correlation value in a case where the component to be measured and each interference component exist alone. As a result, the offset included in the single correlation value is removed, the correlation value is proportional to the concentration of the component to be measured and the interference component, and the measurement error can be reduced. Note that the reference correlation value may not be subtracted.

The concentration calculation unit 64 calculates the concentration of the component to be measured using the plurality of sample correlation values obtained by the correlation value calculation unit 62.

Specifically, the concentration calculation unit 64 calculates the concentration of the component to be measured on the basis of the plurality of sample correlation values obtained by the correlation value calculation unit 62 and the plurality of single correlation values stored in the storage unit 63. More specifically, the concentration calculation unit 64 calculates the concentration of one or more types of components to be measured contained in the sample gas by solving simultaneous equations including a plurality of sample correlation values obtained by the correlation value calculation unit 62, a plurality of single correlation values stored in the storage unit 63, and the concentration of each component to be measured and each interference component.

Next, an example of the operation of the analysis device 100 will be described also as detailed description of each unit. Hereinafter, it is assumed that one component to be measured (for example, $SO_2$) and one interference component (for example, $H_2O$) are contained in the sample gas.

<Reference Measurement>

First, the light source control unit 5 controls each of the laser light sources 2a to 2c to modulate the wavelength of the laser light at the modulation frequency and around the peak of the absorption spectrum of the component to be measured. Before the reference measurement using the span gas, the reference measurement using the zero gas may be performed to measure the reference correlation value.

Next, a span gas (gas having a known component concentration) is introduced into the cell 1 by an operator or automatically, and reference measurement is performed. This reference measurement is performed in each of the span gas in which the component to be measured is present alone and the span gas in which the interference component is present alone.

Specifically, in the reference measurement, the logarithmic arithmetic unit 61 receives the output signal of the photodetector 3 and calculates logarithmic intensity $L(t)$. Then, the correlation value calculation unit 62 calculates a correlation value between the logarithmic intensity $L(t)$ and the two feature signals $F_1(t)$ and $F_2(t)$, and calculates a single correlation value which is a correlation value of each span gas per unit concentration by dividing a value obtained by subtracting the reference correlation value from the correlation value by the concentration of the span gas. Instead of calculating the single correlation value per unit concentration, the relationship between the span gas concentration and the single correlation value of the span gas may be stored.

Specifically, the reference measurement is as follows.

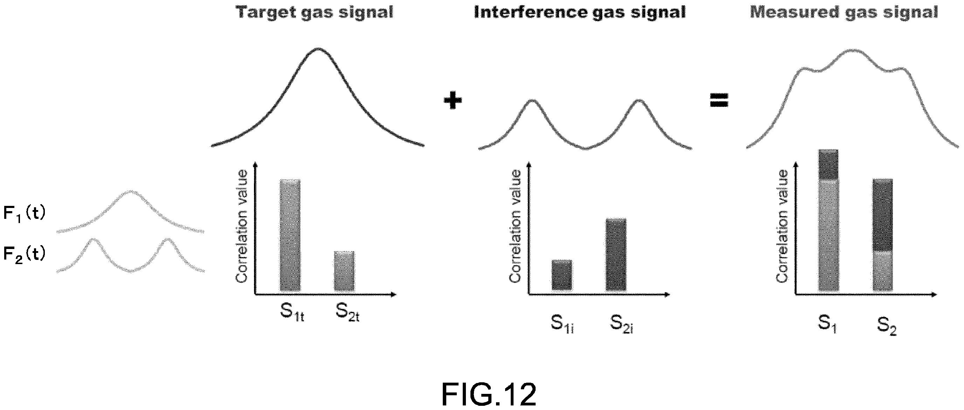

By introducing a span gas in which a component to be measured is present alone into the cell 1, the correlation value calculation unit 62 calculates correlation values $S_{1t}$ and $S_{2t}$ of the component to be measured (see Fig. 12). Here, $S_{1t}$ is a correlation value with the first feature signal, and $S_{2t}$ is a correlation value with the second feature signal. Then, the correlation value calculation unit 62 calculates the single correlation values $s_{1t}$ and $s_{2t}$ by subtracting the reference correlation value $Ri$ from the correlation values $S_{1t}$ and $S_{2t}$ and dividing the result by the span gas concentration ct of the component to be measured. Note that the span gas concentration ct of the component to be measured is input to the signal processing unit 6 in advance by a user or the like.

In addition, by introducing a span gas in which an interference component is present alone into the cell 1, the correlation value calculation unit 62 calculates correlation values $S_{1i}$ and $S_{2i}$ of the interference component (see Fig. 12). Here, $S_{1i}$ is a correlation value with the first feature signal, and $S_{2i}$ is a correlation value with the second feature signal. Then, the correlation value calculation unit 62 calculates the single correlation values $s_{1i}$ and $s_{2i}$ by subtracting the reference correlation value from the correlation values $S_{1i}$ and $S_{2i}$ and dividing the result by the span gas concentration ci of the interference component. Note that the span gas concentration $c_i$ of the interference component is input to the signal processing unit 6 in advance by a user or the like.

The single correlation values sit, $s_{2t}$, $s_{1i}$, and $s_{2i}$ calculated as described above are stored in the storage unit 63. The reference measurement may be performed before product shipment or may be periodically performed.

<Sample Measurement>

The light source control unit 5 controls each of the laser light sources 2a to 2c to modulate the wavelength of the laser light at the modulation frequency and around the peak of the absorption spectrum of the component to be measured.

Next, a sample gas is introduced into the cell 1 by an operator or automatically, and sample is performed.

Specifically, in the sample measurement, the logarithmic arithmetic unit 61 receives the output signal of the photodetector 3 and calculates logarithmic intensity $L(t)$. Then, the correlation value calculation unit 62 calculates sample correlation values between the logarithmic intensity $L(t)$ and the plurality of feature signals $F_1(t)$ and $F_2(t)$, and calculates sample correlation values $S_1'$ and $S_2'$ obtained by subtracting the reference correlation value $Ri$ from the correlation values (see Fig. 12).

Then, the concentration calculation unit 64 solves the following binary simultaneous equations including the sample correlation values $S_1'$ and $S_2'$ calculated by the correlation value calculation unit 62, the single correlation values sit, $s_{2t}$, $s_{1i}$, and $s_{2i}$ in the storage unit 63, and the concentrations $C_{tar}$ and $C_{int}$ of the component to be measured and each interference component.

[Math 2]

$$s_{1t}C_{tar} + s_{1i}C_{int} = S_1'$$
$$s_{2t}C_{tar} + s_{2i}C_{int} = S_2'$$

As a result, the concentration $C_{tar}$ of the component to be measured from which the interference influence has been removed can be determined by a simple and reliable operation of solving the simultaneous equations of the above formulae (Math 2).

Note that, even in a case where it is possible to assume that two or more interference components are present, it is possible to similarly determine the concentration of the component to be measured from which the interference influence has been removed by adding the single correlation value by the number of interference components and solving simultaneous equations having the same number of elements as the number of component types.

That is, in general, in a case where n kinds of gases are present as a total of the component to be measured and the interference component, the following equations (Math 3) are established, where the single correlation value of the k-th gas species in the m-th feature signal is $s_{mk}$, the concentration of the k-th gas species is $C_k$, and the sample correlation value in the m-th feature signal $F_m(t)$ is $S_m'$.

[Math 3]

$$s_{11}C_1 + s_{12}C_2 + s_{13}C_3 + \cdots + s_{1n}C_n = S_1'$$
$$s_{21}C_1 + s_{22}C_2 + s_{23}C_3 + \cdots + s_{2n}C_n = S_2'$$
$$s_{31}C_1 + s_{32}C_2 + s_{33}C_3 + \cdots + s_{3n}C_n = S_3'$$
$$\vdots$$
$$s_{n1}C_1 + s_{n2}C_2 + s_{n3}C_3 + \cdots + s_{nn}C_n = S_n'$$

The concentration of each gas of the component to be measured and the interference component can be determined by solving the n-dimensional simultaneous equations expressed by the equations (Math 3).

According to the analysis device 100 of the present embodiment thus configured, since three different types of semiconductor lasers are used as the laser light sources 2a to 2c, a range of selection of the oscillation wavelengths of the laser light can be increased as compared with a case where a plurality of semiconductor lasers of the same type is used as the laser light sources, and a larger variety of components can be analyzed. In addition, since the laser light sources 2a to 2c are caused to perform pulse oscillation at different timings from each other, measurement by another laser light source becomes possible while a pulse of a certain laser light source is turned off, and a plurality of components can be simultaneously analyzed by a single photodetector. Therefore, the analysis time can be shortened, and the power consumption can be reduced as compared with the case where each laser light source performs continuous wave (CW) oscillation.

Note that the present invention is not limited to the above embodiments.

For example, although the logarithmic arithmetic unit 61 of each of the above embodiments performs logarithmic arithmetic of the light intensity signal of the photodetector 3, the logarithmic arithmetic unit may calculate logarithm (so-called absorbance) of the ratio of the intensity of the sample light to the intensity of the modulated light as the reference light using the light intensity signal of the photodetector 3. At this time, the logarithmic arithmetic unit 61 may calculate the absorbance by calculating the logarithm of the intensity of the sample light, calculating the logarithm of the intensity of the reference light, and then subtracting the logarithm, or may calculate the absorbance by obtaining the ratio between the intensity of the sample light and the intensity of the reference light, and then taking the logarithm of the ratio.

In addition, the correlation value calculation unit 62 of each of the above-described embodiments calculates a correlation value between the intensity-related signal and the feature signal, but may calculate an inner product value of the intensity-related signal and the feature signal.

In each of the above embodiments, the storage unit 63 stores the single correlation value corrected using the reference correlation value. Alternatively, the storage unit 63 may store the single correlation value before correction, and the concentration calculation unit 64 may subtract the reference correlation value from the single correlation value before correction and obtain the corrected single correlation value converted per unit concentration.

The plurality of feature signals are not limited to those in the above embodiment, and may be any functions different from each other. In addition, for example, a function indicating a waveform (sample spectrum) of light intensity, logarithmic intensity, or absorbance obtained by flowing a span gas having a known concentration may be used as the feature signal. When the concentration of one component to be measured is measured, at least one characteristic signal is sufficient.

Furthermore, in a case where n types of gases are present as a total of the component to be measured and the interference component, single correlation values and sample correlation values larger in number than the number of gas types are obtained by using a type of feature signal larger than n, simultaneous equations having an element number larger than the number of gas types are created, and the concentration of each component may be determined by the least squares method. In this manner, it is possible to determine the concentration with a small error even with respect to measurement noise.

The signal processing unit according to the embodiment performs functions of a correlation value calculation unit that calculates a correlation value depending on the concentration of the component to be measured using an intensity-related signal related to the intensity of the sample light and a feature signal from which a predetermined correlation is obtained with respect to the intensity-related signal, and a concentration calculation unit that calculates the concentration of the component to be measured using the correlation value obtained by the correlation value calculation unit. However, other calculation methods may be used.

In the above embodiment, the oscillation wavelength of the laser light source 2 is modulated, but the oscillation wavelength of the laser light source 2 may be fixed.

In the above embodiment, the plurality of laser light sources 2 are pulse-oscillated at the same oscillation cycle, but the oscillation cycles of the laser light sources 2 may be different from each other.

The light source control unit 5 of another embodiment may set the base current (or the base voltage) of the current source (or the voltage source) of the second laser light source 2b to a constant value or 0 without changing the base current (or the base voltage) as illustrated in Fig. 5, or may change the base current of the current source (or the voltage source) of the third laser light source 2c at a predetermined frequency. In this case as well, when the peak value of the pulse current (or pulse voltage) of the current source (or voltage source) of each of the second laser light source 2b and the third laser light source 2c is changed at a predetermined frequency, it is possible to sufficiently modulate the oscillation wavelengths of the second laser light source 2b and the third laser light source 2c by generating a change in the carrier density of the element.

Alternatively, similarly to the control of the first laser light source 2a, the light source control unit 5 may change the base current (or the base voltage) of the current source (or the voltage source) of the second laser light source 2b at a predetermined frequency while keeping the peak value of the pulse current (or the pulse voltage) for pulse oscillation of the current source (or the voltage source) of the second laser light source 2b constant. In this case as well, the oscillation wavelength can be modulated by generating the temperature change of the element of the second laser light source 2b.

Further alternatively, similarly to the control of the first laser light source 2a, the light source control unit 5 may change the base current (or the base voltage) of the current source (or the voltage source) of the third laser light source 2c at a predetermined frequency while keeping the peak value of the pulse current (or the pulse voltage) for pulse oscillation of the current source (or the voltage source) of the third laser light source 2c constant. In this case as well, the oscillation wavelength can be modulated by generating the temperature change of the element of the third laser light source 2c.

In another embodiment, a plurality of some or all of the first laser light source 2a, the second laser light source 2b, and the third laser light source 2c may be provided. The analysis device 100 of another embodiment may not include all of the first laser light source 2a, the second laser light source 2b, and the third laser light source 2c, and may include at least two types selected from the first laser light source 2a, the second laser light source 2b, and the third laser light source 2c.

The third laser light source 2c of the above embodiment is a laser diode that oscillates near-infrared light, but the present invention is not limited thereto. In another embodiment, the third laser light source 2c may be a laser diode that oscillates visible light or ultraviolet light.

In the above embodiment, the concentrations of the plurality of components to be measured contained in the sample gas are measured using the plurality of laser light sources 2, but other measurement items may be measured in addition to the concentration of the components to be measured.

In addition, the sample gas is not limited to the exhaust gas, and may also be the atmosphere, a gas during combustion, a process gas generated in a chemical plant or the like, or may be a liquid or a solid. In this sense, the present invention can be applied to not only a gas but also a liquid or a solid as a component to be measured. In addition, the present invention can be used not only for calculation of the absorbance of the light penetrating through the measurement target but also for calculation of absorbance by reflection.

The same component to be measured may be analyzed using light sources having different oscillation wavelengths. As a result, the amount of information can be increased to further reduce the interference influence.

The photodetector 3 of the above embodiment uses InAsSb as the detection element, but the present invention is not limited thereto. As long as the detection element has sensitivity to the oscillation wavelength of each of the laser light sources 2a to 2c to be used, the light emitted from the plurality of laser light sources 2a to 2c and having passed through the measurement cell 1 can be detected by the single (common) photodetector 3. Examples of such a detection element include HgCdTe and the like in addition to InAsSb. However, since HgCdTe is higher in cost than InAsSb and contains an environmentally hazardous substance, InAsSb is preferably used as the detection element.

In the above embodiment, the light emitted from the plurality of laser light sources 2a to 2c and having passed through the measurement cell 1 is detected by the single photodetector 3, but the present invention is not limited thereto. In another embodiment, a plurality of photodetectors 3 corresponding to the laser light sources 2a to 2c may be provided. In this case, the photodetector 3 does not need to use a quantum photoelectric element excellent in responsiveness, and may use another type such as a relatively inexpensive thermal element, e.g., a thermopile element. In addition, a band pass filter or the like may be provided on the optical path of the laser light transmitted through the measurement cell 1 to divide the wavelength of the laser light, and the laser light may be detected by the plurality of photodetectors 3.

Furthermore, in the above embodiment, the signal separation unit 7 is configured using an analog electric circuit (sample-and-hold circuit 71), but may be configured using a digital electric circuit. In this case, it is conceivable that after the light intensity signal from the photodetector 3 is converted into a digital signal by the AD converter, the digital signal is sampled by a sample signal synchronized with the pulse oscillation of each of the laser light sources 2a to 2c and separated.

Furthermore, in addition to the above embodiment, the signal separation unit 7 may separate an offset signal, which is a signal during the pulse-off period of the plurality of laser light sources 2a to 2c, from the light intensity signal of the photodetector 3. Then, the signal processing unit 6 uses the offset signal to correct the light intensity signal of the photodetector 3 in the reference measurement and the sample measurement. With such a configuration, since the offset signal of the photodetector 3 can also be acquired substantially at the same time as the pulse oscillation, a change in the offset signal due to the disturbance can be captured, whereby the analysis with high accuracy can be performed. In addition, it is not necessary to stop the laser light source 2 in order to acquire the offset signal or to provide a light shielding structure for blocking light entering the photodetector 3.

In addition, the analysis device 100 can be applied to a plurality of types of analyses, for example, may be applied to analyze a plurality of different types of components, or may be applied to analyze the same component separately for low concentration and high concentration, for example.

The analysis device 100 of another embodiment may be configured to oscillate one or more laser light sources other than the quantum cascade laser among the laser light sources 2a to 2c with a continuous wave (CW). For example, the analysis device 100 of another embodiment may be configured to cause the interband cascade laser, which is the second laser light source 2b, and the semiconductor laser, which is the third laser light source 2c, to perform continuous wave (CW) oscillation, and cause only the quantum cascade laser, which is the first laser light source 2a with high power consumption, to perform pulse oscillation. In this case, it is preferable to include a plurality of photodetectors 3 respectively corresponding to the laser light sources 2a to 2c. Even with such a configuration, since a plurality of different types of semiconductor lasers are used as the laser light sources, a range of selection of the oscillation wavelengths of the laser light can be increased as compared with a case where a plurality of semiconductor lasers of the same type is used as the laser light sources, and a larger variety of components can be analyzed. In addition, if a plurality of photodetectors 3 corresponding to the laser light sources 2a to 2c are provided, simultaneous measurement by the laser light sources 2a to 2c becomes possible, and a plurality of components can be analyzed simultaneously. As a result, the analysis time can be shortened, and the power consumption can be reduced. Note that one or more of the laser light sources 2a to 2c may be configured to oscillate a continuous wave (CW).

In addition, various modifications and combinations of the embodiments may be made without departing from the gist of the present invention.

Industrial Applicability

It is possible to analyze many types of components in a short time in an analysis device using a laser light source.

Reference Signs List

100　analysis device
1　measurement cell
2a　first laser light source (QCL)
2b　second laser light source (ICL)
2c　third laser light source (semiconductor laser other than QCL and ICL)
3　photodetector
5　light source control unit

**Claims**

1. An analysis device that irradiates a measurement cell into which a sample has been introduced with light, detects light having passed through the measurement cell, and analyzes a component to be measured contained in the sample, the analysis device comprising:

two or more laser light sources selected from a first laser light source that is a quantum cascade laser, a second laser light source that is an interband cascade laser, and a third laser light source that is a semiconductor laser other than the quantum cascade laser and the interband cascade laser;

a photodetector that detects light emitted from each of the laser light sources and having passed through the measurement cell; and

a light source control unit that causes the laser light sources to perform pulse oscillation at different timings.

2. The analysis device according to claim 1, wherein the light source control unit causes the laser light sources to perform pulse oscillation at a same oscillation cycle.

3. The analysis device according to claim 1 or 2, wherein the light emitted from each of the laser light sources and having passed through the measurement cell is detected by a smaller number of the photodetectors than the number of the laser light sources.

4. The analysis device according to claim 3, wherein the light emitted from each of the laser light sources and having passed through the measurement cell is detected by the single photodetector.

5. The analysis device according to any one of claims 1 to 4, wherein the laser light sources emit laser lights having oscillation wavelengths respectively corresponding to different components to be measured.

6. The analysis device according to any one of claims 1 to 5, wherein the photodetector has sensitivity to an oscillation wavelength of each of the laser light sources.

7. The analysis device according to claim 6, wherein the photodetector uses a quantum photoelectric element.

8. The analysis device according to any one of claims 1 to 7 comprising the first laser light source and the second laser light source or the third laser light source,

wherein the light source control unit modulates an oscillation wavelength of the first laser light source by changing a base current or a base voltage for modulation of the first laser light source at a predetermined frequency, and modulates an oscillation wavelength of the second laser light source or the third laser light source by changing a peak value of a pulse current or a pulse voltage for pulse oscillation of the second laser light source or the third laser light source at a predetermined frequency.

9. The analyzing apparatus according to any one of claims 1 to 8, wherein

the component to be measured contains at least HCl and/or HF, and
the light source control unit

modulates an oscillation wavelength of any one of the laser light sources so as to correspond to an optical absorption spectrum of HCl and
modulates an oscillation wavelength of any one of the laser light sources so as to correspond to an optical absorption spectrum of HF.

10. The analysis device according to claim 9 comprising a concentration calculation unit that calculates a concentration of the component to be measured based on an output signal of the photodetector,

wherein the concentration calculation unit, when the concentration of HCl is measured, calculates the concentration based on absorption of HCl of 3.30 $\mu$m or more and 3.64 $\mu$m or less, and
when the concentration of HF is measured, calculates the concentration based on absorption of HF of 2.39 $\mu$m or more and 2.65 $\mu$m or less.

11. An analysis method that irradiates a measurement cell into which a sample has been introduced with light, detects light having passed through the measurement cell, and analyzes a component to be measured contained in the sample, the analysis device comprising:

causing two or more laser light sources selected from a first laser light source that is a quantum cascade laser, a second laser light source that is an interband cascade laser, and a third laser light source that is a semiconductor laser other than the quantum cascade laser and the interband cascade laser to perform pulse oscillation at different timings; and
detecting light emitted from each of the laser light sources and having passed through the measurement cell by a photodetector.

100

FIG.1

CURRENT CONTROL SIGNAL 　　4　　OUTPUT SIGNAL FROM PHOTODETECTOR

FIG.2

DRIVE CURRENT (VOLTAGE)

PULSE TRAIN

MODULATION SIGNAL

TIME

FIG.3

FIG.4

FIG.5

FIG.6

PULSE WIDTH: 10 TO 100 ns
CYCLE: 1 TO 5 MHz

LIGHT
INTENSITY

FIG.7

FIG.8

FIG.9

71

SW

R

Vin

C

Vout

72

FIG.10

MODULATION CYCLE T

OSCILLATION WAVELENGTH

LIGHT INTENSITY I(t)

log

LOGARITHMIC INTENSITY L(t)

×

FEATURE SIGNAL F$_i$(t)

∫

CORRELATION VALUE S$_i$

Time

FIG.11

FIG.12

EP 4 446 728 A1

## INTERNATIONAL SEARCH REPORT

| International application No. |
| --- |
| **PCT/JP2022/044356** |

| A. | CLASSIFICATION OF SUBJECT MATTER |
| --- | --- |

*G01N 21/01*(2006.01)i; *G01N 21/3504*(2014.01)i
FI:   G01N21/3504; G01N21/01 D

According to International Patent Classification (IPC) or to both national classification and IPC

| B. | FIELDS SEARCHED |
| --- | --- |

Minimum documentation searched (classification system followed by classification symbols)

G01N21/00-21/61

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2017-156304 A (TOSHIBA CORP.) 07 September 2017 (2017-09-07)<br>paragraphs [0002]-[0006], [0012]-[0021], fig. 1, 5, 6 | 1-11 |
| Y | JP 2014-94355 A (MITSUBISHI HEAVY IND., LTD.) 22 May 2014 (2014-05-22)<br>paragraph [0082] | 1-11 |
| Y | JP 2018-521330 A (NEO MONITOR AS) 02 August 2018 (2018-08-02)<br>claims 9, 17, paragraphs [0003], [0077], [0080] | 1-11 |
| Y | US 2020/0166453 A1 (TECHNISCHE UNIVERSITAET WIEN) 28 May 2020 (2020-05-28)<br>paragraph [0055] | 1-11 |
| Y | JP 2019-66477 A (HORIBA, LTD.) 25 April 2019 (2019-04-25)<br>paragraph [0025] | 7 |
| Y | JP 2004-85252 A (HORIBA, LTD.) 18 March 2004 (2004-03-18)<br>paragraph [0012] | 9-10 |
| Y | JP 7-277720 A (SUMITOMO SITIX CORP.) 24 October 1995 (1995-10-24)<br>paragraphs [0014], [0015] | 9-10 |

☑ Further documents are listed in the continuation of Box C.     ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 January 2023** | **07 February 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| | International application No. |
|---|---|
| | **PCT/JP2022/044356** |

**C. DOCUMENTS CONSIDERED TO BE RELEVANT**

| Category* | Citation of document, with indication, where appropriate, of the relevant passages | Relevant to claim No. |
|---|---|---|
| Y | JP 2001-311686 A (SHOWA DENKO KK) 09 November 2001 (2001-11-09)<br>paragraph [0024] | 9-10 |
| Y | US 5606419 A (NORSK HYDRO A.S.) 25 February 1997 (1997-02-25)<br>fig. 1c, column 2, lines 25-41 | 9-10 |

Form PCT/ISA/210 (second sheet) (January 2015)

INTERNATIONAL SEARCH REPORT
Information on patent family members

International application No.

**PCT/JP2022/044356**

| Patent document cited in search report | | | Publication date (day/month/year) | Patent family member(s) | | | Publication date (day/month/year) |
|---|---|---|---|---|---|---|---|
| JP | 2017-156304 | A | 07 September 2017 | (Family: none) | | | |
| JP | 2014-94355 | A | 22 May 2014 | (Family: none) | | | |
| JP | 2018-521330 | A | 02 August 2018 | US | 2018/0172580 | A1 | |
| | | | | claims 9, 17, paragraphs [0003], [0098], [0101] | | | |
| | | | | WO | 2016/200274 | A1 | |
| | | | | EP | 3308143 | A1 | |
| | | | | KR | 10-2018-0017131 | A | |
| | | | | CN | 107850535 | A | |
| | | | | CA | 2989119 | A1 | |
| US | 2020/0166453 | A1 | 28 May 2020 | WO | 2019/018870 | A1 | |
| | | | | EP | 3658896 | A1 | |
| | | | | CA | 3070020 | A1 | |
| JP | 2019-66477 | A | 25 April 2019 | US | 2019/0101491 | A1 | |
| | | | | paragraph [0036] | | | |
| | | | | EP | 3467477 | A1 | |
| | | | | CN | 109596538 | A | |
| JP | 2004-85252 | A | 18 March 2004 | (Family: none) | | | |
| JP | 7-277720 | A | 24 October 1995 | (Family: none) | | | |
| JP | 2001-311686 | A | 09 November 2001 | US | 2002/0051132 | A1 | |
| | | | | paragraph [0149] | | | |
| | | | | CN | 1330267 | A | |
| | | | | CN | 1683923 | A | |
| US | 5606419 | A | 25 February 1997 | WO | 94/11713 | A1 | |
| | | | | EP | 670035 | A1 | |
| | | | | AU | 5578494 | A | |
| | | | | CA | 2149663 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019066477 A